# FASCICULE DE BREVET EUROPEEN

(11) **EP 0 380 393 B1**
(45) Date de publication et mention de la délivrance du brevet: **06.04.1994**
(21) Numéro de dépôt: 90400162.5
(22) Date de dépôt: 22.01.1990
(51) Int. Cl.: C07C 69/63, C07C 67/307

(54) **Preparation d'esters d'acide-2 chloropropionique**
Herstellung von 2-Chlorpropionsäureester
Preparation of 2-chloropropionic-acid esters

(30) Priorité: 23.01.1989 FR 8900983
(43) Date de publication de la demande: 01.08.1990
(73) Titulaire: RHONE-POULENC CHIMIE, 92408 Courbevoie Cédex (FR)
(72) Inventeur: Desmurs, Jean-Roger, F-69360 - St Syphorien d'Ozon (FR); Metivier, Pascal, F-69001 Lyon (FR); Padilla, Geneviève, F-69360 Solaize (FR); Rajoharison, Harivelo, F-38130 Echirolles (FR)
(74) Mandataire: Dutruc-Rosset, Marie-Claude

(56) Documents cités:
- EP-A- 0 163 435
- CH-A- 397 631
- CH-A- 410 918

## Description

L'invention se rapporte à un procédé de préparation d'esters d'acides α-chlorocarboxyliques optiquement actifs à partir d'esters d'acides α-hydroxycarboxyliques correspondant, notamment d'esters d'acide 2-chloropropionique optiquement actifs à partir d'esters lactiques optiquement actifs correspondant. Ces composés sont des intermédiaires chimiques bien connus permettant d'obtenir des produits chimiques utiles notamment comme herbicides. L'intérêt de préparer des herbicides optiquement actifs réside dans le fait que ceux-ci sont actifs à des doses moitié moindre que les composés racémiques correspondants. Il en découle un intérêt majeur en ce qui concerne notamment la sauvegarde de l'environnement.

De nombreux procédés ont déjà été proposés afin de réaliser cette synthèse avec les rendements les plus élevés possibles.
Ainsi le brevet français FR-B-2459221 décrit la chloration de l'ester lactique d'alcoyle racémique ou optiquement actif en présence de chlorure de thionyle et d'une base organique en maintenant dans le mélange réactionnel un excès molaire de chlorure de thionyle d'au moins 2,5% par rapport à la quantité de lactate d'alcoyle introduit dans le mélange, à une température maintenue inférieure à la température de décomposition du chlorosulfite de lactate d'alcoyle pendant cette première étape puis en chauffant dans une deuxième étape le mélange réactionnel résultant de la première étape à une température au moins égale à la température de décomposition du chlorosulfite de lactate d'alcoyle.

Par le document EP-A-0163435 on connait la chloration du lactate d'alkyle par le phosgène en présence d'une amine tertiaire. Cette réaction passe par un intermédiaire chloroformiate de formule CH₃-CH(OCOCl)-CO₂R qui est ensuite décomposé.

Néammoins ce procédé nécéssite l'utilisation d'une quantité importante d'amine (quantité stoechiometrique) et implique l'élimination subséquente de celle ci. Elle requiert, par ailleurs, un gros éxcès de COCl₂. Par DE-A- 1135893 il est connu de faire réagir du 1-butyne 3-ol avec du phosgène en éxcès et en présence d'environ 10 % de dimethylformamide à une température comprise entre 50 et 55°c pour conduire au composé chloré correspondant avec un rendement de 79 %. Toutefois, ce document ne donne aucun enseignement en ce qui concerne l'isomérie optique pour la simple raison qu'il s'agit dans ce document de composés racémiques.

Au vu de l'art antérieur qui vient d'être analysé un premier objet de l'invention est de proposer un autre procédé permettant d'aboutir, notamment en partant d'un ester d'acide lactique. optiquement actif, à un ester d'acide 2-chloropropionique avec un excellent rendement tout en conservant une excellente activité optique (rendement optique).

Un autre objet de l'invention est de proposer un procédé simple notamment au niveau de la phase de retraitement.

Ainsi l'invention a pour object un procédé de préparation de l'ester de l'acide α -chlorocarboxylique optiquement actif de formule : 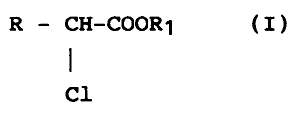
- R est un radical alkyle C₁-C₁₈ linéaire ou ramifié,
- R₁ étant un reste hydrocarbyle éventuellement substitué à partir d'un α hydroxy carboxylate actif correspondant de formule 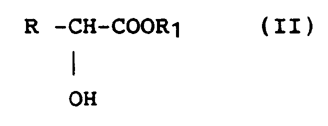 ledit procédé étant caractérisé en ce que l'on met en contact ledit carboxylate de formule (II) avec du phosgène et un composé choisi parmi les amides aliphatiques, les lactames aliphatiques et les urées aliphatiques.

Le groupement R₁ est notamment choisi parmi les radicaux suivants :
C₁-C₁₈ alkyle linéaire ou ramifié, de préférence C₁-C₁₂
C₂-C₁₈ alcényle linéaire ou ramifié, de préférence C₂-C₁₂
C₂-C₁₈ alcynyle linéaire ou razmifié, de préférence C₂-C₁₂
C₃-C₁₈ cycloalkyle linéaire ou ramifié, de préférence C₃-C₁₂
C₆-C₁₄ aryle linéaire ou ramifié, de préférence C₆-C₁₀
C₇-C₁₅ aralkyle linéaire ou ramifié, de préférence C₇-C₁₁,
ces radicaux étant éventuellement substitués par un ou plusieurs atomes d'halogène, radicaux C1-C6 alcoxy ou alkylthio, les radicaux aryle ou aralkyle pouvant, en outre, comporter en remplacement d'un à quatre atomes de carbone, respectivement un à quatre hétéroatomes choisis parmi l'atomes d'oxygène, de soufre, d'azote, (par éxemple furyle, thiophényle, piridyle). De préférence R₁ est un radical C₁-C₆ alkyle. Parmi les amides on peut citer ceux répondant à la formule :

R₂CO-NR₃R₄ (III)

dans laquelle :
R₂ est un atome d'hydrogène, un groupement C₁ C₆ alkyle, C₃-C₁₀ cycloalkyle où un à trois atomes de carbone peuvent être remplacés par respectivement 1 à 3 hétéroatomes choisis parmi les atomes d'oxygène, soufre, ou d'azote, R₃,R₄ identiques ou differents sont un groupement C₁-C₆ alkyle, C₃-C₁₀ cycloalkyle où un à trois atomes de carbone peuvent ètre remplacés par respectivement 1 à 3 hétéroatomes choisis parmi les atomes d'oxygène, de soufre, ou d'azote.

Par exemple parmi les amides convenant pour la présente invention ou peut citer la N,N-dimethyl formamide, N-N diméthylacétamide.

Parmi les lactames utilisables selon le procédé de l'invention on peut citer ceux de formule : 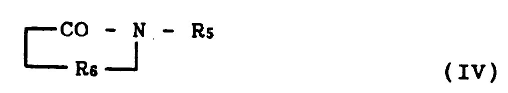
dans laquelle R₅ est un groupement C1-C6 alkyle, C3-C10 cycloalkyle où un à trois atomes de carbone peuvent être remplacés par respectivement un à trois hétéroatomes choisis parmi les atomes d'oxygène, de soufre, ou d'azote.
R₆ est un groupement C₂-C₁₀ alkylène où un à trois atomes de carbone peuvent être remplacés respectivement par un à trois hétéroatomes choisis parmi l'atome d'oxygène, de soufre ou d'azote.

Par exemple parmi les lactames pouvant convenir pour la présente invention on peut citer en particulier : N-méthyl - pyrrolidinone.

Parmi les urées utilisables dans le procédé selon l'invention on peut citer celles répondant à la formule. 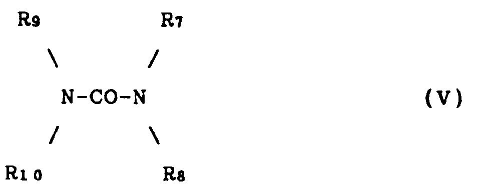
R₇, R₈, R₉, R₁₀ identiques ou différents representant un atome d'hydrogène, un groupement C₁-C₆ alkyle, C₃-C₁₀ cycloalkyle où un à trois atomes de carbone peuvent être remplacés par respectivement 1 à 3 hétéroatomes choisis parmi les atomes d'oxygène, de soufre ou d'azote. Par exemple parmi les urées convenant pour la présente invention on peut citer : la tetraméthyl - urée;

Parmi les trois catégories de composés énumérées ci-avant on préfèrera les amides ou les lactames.La réaction peut être effectuée en masse, ainsi il a été montré que le produit final, tel que par exemple le(s) 2-chloropropionate(s) donnent des résultats particulièrment satisfaisants, ce qui permet d'utiliser en continu le procédé par introduction des réactifs et purges de l'ester α chlorocarboxylique formé ou en présence d'un solvant protique ou aprotique. Parmi les solvants aprotiques on peut citer les hydrocarbures aliphatiques saturés tels que le n-pentane, l'isopentane, le 2- méthylhexane, le 2,2,5-triméthylhexane, les hydrocarbures aromatiques tels que le benzène, le toluène, l'éthylbenzène, les éthers aliphatiques saturés tels que le tétrahydrofuranne, l'isopentyléther, les éthers aromatiques tels que le benzythyléther, les cétones aliphatiques saturées ou aromatiques comme la méthyl-éthylcétone, la méthylisobutyl-cétone, l'acétophénone, les hydrocarbures halogènés saturés aliphatiques ou aromatiques comme les mono ou polyhalobenzène, le 1-chloro- 2méthylpropane, le chlorure d'isobutyle, les esters aliphatiques saturés ou aromatiques comme l'isobutyle, l'acétate d'éthyle, le benzoate de méthyle. Tous ces solvants peuvent être présents seuls ou en mélange.

Soulignons que la nomenclature employée est française mis à part le fait que la place des substituants est indiquée en amont desdits substituants. Parmi ces solvants on préfère les hydrocarbures aromatique halogénés ou non. Si la réaction s'effectue en présence d'un solvant, la dilution du produit réactionnel sera notamment comprise entre 0,5 % et 99 % en poids par rapport au total de la solution et de préférence entre 5 et 50 %.

La proportion molaire de COCl₂ par rapport au lactate peut varier dans un intervalle considérable bien que l'homme de métier comprendra aisément que le rapport COCl₂/lactate est au moins proche de 1 afin que la réaction soit complète.

Néammoins un rapport molaire COCl₂/lactate compris entre 0,9 et 3 sera préfèré et avantageusement on choisira un rapport compris entre 0,95 et 1,5 et de façon très avantageuse entre 1 et 1,2. En ce qui concerne les amides, lactames, et urées on emploiera une quantité telle que le rapport molaire par rapport au COCl₂ sera comprise entre 1/100 et 1/2 ; de préférence inférieure à 1/5. La température de réaction est avantageusement comprise entré 0°C et 100°C.

Selon une autre variante avantageuse l'utilisation d'un solvant et d'un amide, urée lactame à point d'ébulition élevé par rapport au chloropropionate de C₁-C₃ alkyle obtenu, permet la récupération du solvant et de l'amide, lactame ou urée en pied de réacteur et le recyclage de ce dernier.
Le procédé de l'invention peut être aisément mis en oeuvre en continu.

Les exemples ci-après illustrent l'invention sans toutefois la limiter.

### CHLORATION DU LACTATE DE METHYLE PAR COCl₂EN PRESENCE DE DMF

Dans un tricol de 50 ml, on charge 30 ml de chlorobenzène, 6,05 g (58,2 mMoles) de R-(+)-Lactate de méthyle (pureté optique 96,7 **%**) et 450 ul de diméthylformamide,

On chauffe le mélange à 80°C puis on additionne à cette température 6,32g (63,8 mMoles) de COCl₂ pendant 1,33 h. On maintient le mélange réactionnel à 80°C pendant 1 heure puis on refroidi rapidement à température ambiante.

On obtient un mélange réactionnel brut de coloration rouge foncé contenant 6,6 g de S-(-)-chloropropionate de méthyle (rendement 92,5 %) d'après le dosage en chromatographie en phase vapeur en présence de dichloro-1,2 benzène comme étalon interne.

Ce mélange réactionnel est hydrolysé par 20 ml d'eau. La phase aqueuse est décantée puis lavée une fois avec CH₂Cl₂. Les couches organiques réunies sont lavées trois fois à l'eau puis séchées sur NA₂SO₄ pendant une nuit.

Une partie aliquote de la phase organique totale est prélevée puis distillée sous vide. On élimine la fraction distillant à 33°C/30 mbars.

L'analyse de cette dernière fraction en chromatographie phase vapeur à l'aide d'une colonne chirale montre que le S-(-)-chloropropionate de méthyle obtenu contient 3,2 % de l'énantiomère dextrogyre le R-(+)-chloropropionate de méthyle (rendement optique ≈ 100 %)

### CHLORATION DU LACTATE D'ISOBUTYLE PAR COCl₂EN PRESENCE DE DMF

Dans un tricol de 50 ml, on charge 30 ml de chlorobenzène, 9,36 g (64,1 mMoles) de R-(+)-Lactate d'isobutyle (α_{d} =+14,1, pureté optique 97,9 %) et 490 ul de diméthylformamide.

On chauffe le mélange à 80°C puis on additionne à cette température 6,98 g (70,5 mmoles) de COCl₂ pendant 2,83 heures. On maintient le mélange réactionnel à 80°C 1 heure puis refroidi rapidement à température ambiante.

On obtient un mélange réactionnel brut de coloration jaune orange foncé contenant 9,44 g de S-(-)-chloropropionate d'isobutyle (rendement 90 %) d'après le dosage en chromatographie en phase vapeur en présence de dichloro-1,2 benzène comme étalon interne.

Ce mélange réactionnel est hydrolysé par 20ml d'eau. La phase aqueuse est décantée puis lavée une fois avec CH₂Cl₂. Les couches organiques réunies sont lavées deux fois à l'eau puis séchées sur Na₂SO₄ pendant une nuit.

Le dichlorométhane est chassé sous vide à température ambiante et le dichlorobenzène à 29°C sous 22 mbar. La fraction de tête distillant entre 20°C et 43,5°C sous 5 mbar est éliminée et la fraction de coeur distillant à 44-45,5°C sous 5 mbar est analysée en chromatographie phase vapeur sur une colonne contenant une phase stationnaire chirale. Cette analyse montre que le S-(-)-chloropropionate d'isobutyle obtenu contient 2,9 % de l'énantiomètre dextrogyre (R-(+)-chloropropionate d'isobutyle)
(rendement optique ≈ 96 %)

### CHLORATION DU LACTATE D'ISOBUTYLE PAR COCl₂EN PRESENCE DE DMF

Dans un tricol de 50 ml, on charge 30 ml de chlorobenzène, et 470 ul de diméthylformamide. On chauffe le mélange à 80°C puis on additionne en parallèle à cette température 8,82 g (60,4 mMoles) de R-(+)-Lactate d'isobutyle et 6,66 g (67,3 mmoles) de COCl₂ pendant 1,17 heure. On maitient le mélange réactionnel à 80°C pendant 1 heure puis rapidement à température ambiante.

On obtient un mélange réactionnel brut de coloration jaune orange foncé contenant 9,54 g de S-(-)-chloropropionate d'isobutyle (rendement 96 %) d'après le dosage en chromatographie en phase vapeur en présence de dichloro-1,2 benzène comme étalon interne.

Ce mélange réactionnel est hydrolysé par 20ml d'eau. La phase aqueuse est décantée puis lavée une fois avec CH₂Cl₂. Les couches organiques réunies sont lavées deux fois à l'eau puis séchées sur Na₂SO₄ pendant une nuit.

Le dichlorométhane est chassé sous vide à température ambiante et le chlorobenzène à 29°C sous 22 mbar. La fraction de tête distillant entre 20°C et 43,5°C sous 5 mbar est éliminée et la fraction de coeur distillant à 44-45,5°C sous 5 mbar est analysée en chromatographie phase vapeur sur une colonne contenant une phase stationnaire chirale. Cette analyse montre que le S-(-)-chloropropionate d'isobutyle obtenu contient 2,7 % de l'énantiomètre dextrogyre (R-(+)-chloropropionate d'isobutyle).
(rendement optique ≈ 99 %)

### CHLORATION DU LACTATE D'ISOBUTYLE PAR COCl₂EN PRESENCE DE DMF

Dans un tricol de 50 ml, on charge 30 ml de trichloro-1,2,4 benzène et 480 µl de diméthylformamide. On chauffe le mélange à 80°C puis on additionne en parallèle à cette température 9,01 g (61,7 mMoles) de R-(+)-Lactate d'isobutyle et 6,86 g (67,3 mMoles) de COCl₂ pendant une heure. On maintient le mélange réactioonel 80°C pendant une heure puis refroidi rapidement à température ambiante.

On obtient un mélange réactionnel brut de coloration jaune orange foncé contenant 9,23 g de S-(-)-chloropropionate d'isobutyle (rendement 91 %) d'après le dosage en chromatographie en phase vapeur en présence de chlorobenzène comme étalon interne.

Ce mélange réactionnel est hydrolysé par 20ml d'eau. La phase aqueuse est décantée puis lavée une fois avec CH₂Cl₂. Les couches organiques réunies sont lavées deux fois à l'eau puis séchées sur Na₂SO₄ pendant une nuit.

Le dichlorométhane est chassé sous vide à température ambiante.

La fraction de tête distillant entre 20°C et 43,5°C sous 5 mbar est éliminée et la fraction de coeur distillant à 44-45,5°C sous 5 mbar est analysée en chromatographie phase vapeur sur une colonne contenant une phase stationnaire chirale. Cette analyse montre que le S-(-)-chloropropionate d'isobutyle obtenu contient 2,7 % de l'énantiomètre dextrogyre (R-(+)-chloropropionate d'isobutyle).
(rendement optique ≈ 99 %).

### CHLORATION DU LACTATE D'ISOBUTYLE PAR COCl₂EN PRESENCE DE N,N-DIBUTYLFORNAMIDE

Dans un tricol de 50 ml, on charge 28 ml de tricloro-1,2,4 benzène et 1,05 ml de dibutylformamide. On chauffe le mélange à 80°C puis on additionne en parallèle à cette température 8,39 g (57,5 mMoles) de R-(+)-Lactate d'isobutyle et 6,26 g (63,2 mMoles) de COCl₂ pendant une heure. On maintient le mélange réactionnel à 80°C pendant une heure puis refroidi rapidement à température ambiante.

On obtient un mélange réactionnel brut de coloration marron rouge foncé contenant 8,74 g de S-(-)-chloropropionate d'isobutyle (rendement 92 %) d'après le dosage en chromatographie en phase vapeur en présence de chlorobenzène comme étalon interne.

Ce mélange réactionnel est hydrolysé par 20ml d'eau. La phase aqueuse est décantée puis lavée une fois avec CH₂Cl₂. Les couches organiques réunies sont lavées deux fois à l'eau puis séchées sur Na₂SO₄ pendant une nuit.

Le dichlorométhane est chassé sous vide à température ambiante.

La fraction de tête distillant entre 20°C et 43,5°C sous 5 mbar est éliminée et la fraction de coeur distillant à 44-45,5°C sous 5 mbar est analysée en chromatographie phase vapeur sur une colonne contenant une phase stationnaire chirale. Cette analyse montre que le S-(-)-chloropropionate d'isobutyle obtenu contient 2,9 % de l'énantiomètre dextrogyre (R-(+)-chloropropionate d'isobutyle).
(rendement optique ≈ 98 %)

### CHLORATION DU LACTATE D'ISOBUTYLE PAR COCl₂EN PRESENCE DE : DMAC (Addition de phosgène sur une solution de lactate+DMAC+Solvant)

Dans un tricol de 50 ml, on charge 30 ml de chlorobenzène, 8,74 g (60 mMoles) de R-(+)-Lactate d'isobutyle (^{a}d = + 14,1 , pureté optique 97,9 %) et 550 µl de Diméthylacetamide. On chauffe le mélange à 80°C puis on additionne à cette température 6,52 g (65,8 mMoles) de COCl₂ pendant 1,15 h. On maintient le mélange réactionnel à 80°C pendant 1 h puis refroidi rapidement ambiante.

On obtient un mélange réactionnel brut de coloration jaune orange foncé contenant 4,20 g de S-(-)-chloropropionate d'isobutyle (rendement 43,3 %) d'après le dosage en chromatographie en phase vapeur en présence de dichloro-1,2 benzène comme étalon interne.

Ce mélange réactionnel est hydrolysé par 20 ml d'eau. La phase aqueuse est décantée muis lavée une fois avec CH₂Cl₂. Les couches organiques réunies sont lavées deux fois à l'eau puis séchées sur Na₂SO₄ pendant 1 nuit.

Le dichlorométhane est chassé sous vide à température ambiante et le chlorobenzène à 29°C sous 22 mbars. La fraction de tête distillant entre 20°C et 43,5°C sous 5 mbars est éliminée et la fraction de coeur distillant à 44-45,5°C sous 4 mbars est analysée en chromatographie phase vapeur sur une colonne contenant une phase stationnaire chirale. Cette analyse montre que le S-(-)-chloropropionate d'isobutyle obtenu contient 3,5 % de l'énantiomère dextrogyre (R-(+)-chloropropionate d'isobutyle).
(rendement optique ≈ 97 %).

### CHLORATION DU LACTATE D'ISOBUTYLE PAR COCl₂EN PRESENCE DE NMP (Addition de phosgène sur une solution de lactate+NMP+Solvant).

Dans un tricol de 50 ml, on charge 30 ml de chlorobenzène, 9,1 g (62,3 mMoles) de R-(+)-Lactate d'isobutyle (^{a}d = + 14,1 , pureté optique 97,9 %) et 600 µl de N-méthyl pyrrolidone. On chauffe le mélange à 80°C puis on additionne à cette température 6,79 g (69 mMoles) de COCl₂ pendant 1,15 h. On maintient le mélange réactionnel à 80°C pendant 1 h puis refroidi rapidement ambiante.

On obtient un mélange réactionnel brut de coloration jaune orange foncé contenant 7,21 g de S-(-)-chloropropionate d'isobutyle (rendement 70 %) d'après le dosage en chromatographie en phase vapeur en présence de dichloro-1,2 benzène comme étalon interne.

Ce mélange réactionnel est hydrolysé par 20 ml d'eau. La phase aqueuse est décantée muis lavée une fois avec CH₂Cl₂. Les couches organiques réunies sont lavées deux fois à l'eau puis séchées sur Na₂SO₄ pendant 1 nuit.

Le dichlorométhane est chassé sous vide à température ambiante et le chlorobenzène à 29°C sous 22 mbars. La fraction de tête distillant entre 20°C et 43,5°C sous 5 mbars est éliminée et la fraction de coeur distillant à 44-45,5°C sous 4 mbars est analysée en chromatographie phase vapeur sur une colonne contenant une phase stationnaire chirale. Cette analyse montre que le S-(-)-chloropropionate d'isobutyle obtenu contient 2,3 % de l'énantiomère dextrogyre (R-(+)-chloropropionate d'isobutyle).
(rendement optique ≈ 99 %).

### CHLORATION DU LACTATE D'ISOBUTYLE PAR COCL₂EN PRESENCE DE TMU (Addition de phosgène sur une solution de lactate+TMU+Solvant

Dans un tricol de 50 ml, on charge 30 ml de chlorobenzène, 10,95 (75 mMoles) de R-(+)-Lactate d'isobutyle (^{a}D = + 14,1 , pureté optique 97,9 %) et 900 µl de tétraméthylurée (TMU). On chauffe le mélange à 80°C puis on additionne à cette température 8,17 g (82,5 mMoles) de COCl₂ pendant 1,5 h. On maintient le mélange réactionnel à 80°C pendant 1 h puis refroidi rapidement ambiante.

On obtient un mélange réactionnel brut de coloration jaune orange foncé contenant 1,66 g de S-(-)-chloropropionate d'isobutyle (rendement 13,4 %) d'après le dosage en chromatographie en phase vapeur en présence de dichloro-1,2 benzène comme étalon interne.

Ce mélange réactionnel est hydrolysé par 20 ml d'eau. La phase aqueuse est décantée puis lavée une fois avec CH₂Cl₂. Les couches organiques réunies sont lavées deux fois à l'eau puis séchées sur Na₂SO₄ pendant 1 nuit.

Le dichlorométhane est chassé sous vide à température ambiante et le chlorobenzène à 29°C sous 22 mbars. La fraction de tête distillant entre 20°C et 43,5°C sous 5 mbars est éliminée et la fraction de coeur distillant à 44-45,5°C sous 4 mbars est analysée en chromatographie phase vapeur sur une colonne contenant une phase stationnaire chirale. Cette analyse montre que le S-(-)-chloropropionate d'isobutyle obtenu contient 5,9 % de l'énantiomère dextrogyre (R-(+)-chloropropionate d'isobutyle).
(rendement optique ≈ 92 %).

### CHLORATION DU LACTATE D'ISOBUTYLE PAR COCl₂EN PRESENCE DE N-formyl-morpholine.

Dans un tricol de 50 ml, on charge 30 ml de trichloro 1, 2, 4 benzene ainsi de 0,86 ml de N-formyl morpholine. On chauffe le mélange à 80°C puis on additionne en parallèle à cette température 12,54 g (85,9 mMoles) de R-(+)-Lactade d'isobutyle et 9,34 g (94,3 mMoles) de COCl₂ pendant 1,05 h. On maintient le mélange réactionnel à 80°C pendant 1 h puis on refroidi rapidement à température ambiante.

On obtient un mélange réactionnel brut de coloration jaune orange foncé contenant 11,78 g de S-(-)-chloropropionate d'isobutyle (rendement 96 %) d'après le dosage en chromatographie en phase vapeur en présence de dichloro-1,2 benzène comme étalon interne.

Une fraction du mélange réactionnel est distillée sous vide. La fraction de tête distillant entre 20°C et 43,5°C sous 5 mbars est éliminée et la fraction de coeur distillant à 44-45,5°C sous 5 mbars est analysée en chromatographie phase vapeur sur une colonne contenant une phase stationnaire chirale. Cette analyse montre que le S-(-)-chloropropionate d'isobutyle obtenu contient 3,3 % de l'énantiomère dextrogyre (R-(+)-chloropropionate d'isobutyle).
(rendement optique ≈ 98 %).

## Revendications

1. Procédé de préparation de l'ester d'acide α-chlorocarboxylique optiquement actif de formule 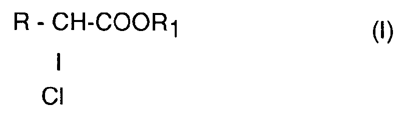 à partir de l'ester d'acide α-hydroxycarboxylique optiquement actif correspondant de formule 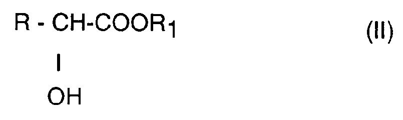 dans les formules (I) et (II) :
- R est un radical alkyle C₁-C₁₈ linéaire ou ramifié,
- R₁ étant un reste hydrocarbylé éventuellement substitué,
ledit procédé étant caractérisé en ce que l'on met en contact ledit ester (II) avec du phosgène et un composé choisi parmi les amides aliphatiques, les lactames aliphatiques et les urées aliphatiques.

2. Procédé selon la revendication 1 caractérisé en ce que R est un radical alkyle C1-C12 linéaire ou ramifié.

3. Procédé selon l'une des revendications 1 et 2 caractérisé en ce que R1 est choisi parmi les radicaux suivants :
C₁-C₁₈ alkyle linéaire ou ramifié, de préférence C₁-C₁₂
C₂-C₁₈ alcényle linéaire ou ramifié, de préférence C₂-C₁₂
C₂-C₁₈ alcynyle linéaire ou ramifié, de préférence C₂-C₁₂
C₃-C₁₈ cycloalkyle linéaire ou ramifié, de préférence C₃-C₁₂
C₆-C₁₄ aryle linéaire ou ramifié, de préférence C₆-C₁₀
C₇-C₁₅ aralkyle linéaire ou ramifié, de préférence C₇-C₁₁,
ces radicaux étant éventuellement substitués par un ou plusieurs atomes d'halogènes, radicaux C_{1-C6} alcoxy ou alkylthio, les radicaux aryle ou aralkyle pouvant, en outre, comporter en remplacement d'une à quatre atomes de carbone, un à quatre hétéroatomes choisis parmi l'atome d'oxygène, de soufre, d'azote (par exemple furyle, thiophényle, piridyle)

4. Procédé de préparation selon la revendication 2, caractérisé en ce que R₁ est un radical C₁-C₆ alkyle et que R est un radical C₁-C₆ alkyle de préférence méthyle.

5. Procédé de préparation selon la revendication 1, caractérisé en ce que :
a) les amides correspondent à la formule :
R₂ CO-NR₃R₄ (III)
dans laquelle R₂ est un atome d'hydrogène, un groupement C₁-C₆ alkyle, C₃-C₁₀ cycloalkyle où un à trois atomes de carbone peuvent être remplacés par respectivement 1 à 3 hétéroatomes choisis parmi les atomes d'oxygène, de soufre, ou d'azote, R₃, R₄ identiques ou différents sont :
un groupement C₁-C₆ alkyle, C₃-C₁₀ cycloalkyle où un à trois atomes de carbone peuvent être remplacés par respectivement un à trois hétéroatomes choisis parmi les atomes d'oxygène, de soufre, ou d'azote.
b) les lactames correspondant à la formule : 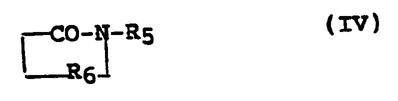 dans laquelle R₅ est un groupement C1-C6 alkyle, C3-C10 cycloalkyle où un à trois atomes de carbone peuvent être remplacés par respectivement un à trois hétéroatomes choisis parmi les atomes d'oxygène, de soufre, ou d'azote et R₆ est un groupement C₂-C₁₀ alkylène ou un à trois atomes de carbone peuvent être remplacés respectivement par un à trois hétéroatomes choisis parmi l'atome d'oxygène, de soufre ou d'azote.
c) les urées correspondent à la formule : 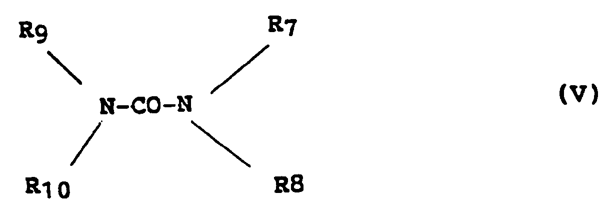 R₇, R₈, R₉, R₁₀ identiques ou différents representants un atome d'hydrogène, un groupement C₁-C₆ alkyle, C₃-C₁₀ où un à trois atomes de carbone peuvent être remplacés par respectivement un à trois hétéroatomes choisis parmi les atomes d'oxygène, de soufre ou d'azote.

6. Procédé de préparation selon la revendication 1, caractérisé en ce que la réaction est effectuée en milieu solvant de préférence un solvant aromatique halogène ou non.

7. Procédé de préparation selon la revendication 1, caractérisé en ce que le rapport molaire.
COCl₂/lactate est compris entre 0,9 et 3 de préférence 0,95 et 1,5.

8. Procédé de préparation selon la revendication 1, caractérisé en ce que le rapport molaire amides, lactames, urées/COCl₂ est compris entre 1/100 et 1/2 de préférence inférieur à 1/5.

9. Procédé selon la revendication 1 caractérisé par le fait que la réaction a lieu en masse et en continu.

10. Procédé selon la revendication 1 caractérisé par le fait que la température de la réaction est comprise entre 0°C et 100°C, de préférence aux environs de 80°C.

## Claims

1. A process for the preparation of the optically active ester of α-chlorocarboxylic acid of the following formula: 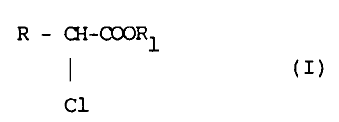 from the corresponding optically active ester of α-hydroxycarboxylic acid of the following formula: 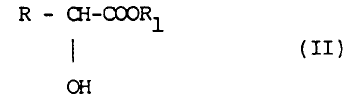 in the formulae (I) and (II):
- R is a branched or straight chain C₁-C₁₈ alkyl radical, and
- R₁ being an optionally substituted hydrocarbyl residue, said process being characterised by bringing said ester (II) into contact with phosgene and a compound selected from aliphatic amides, aliphatic lactams and aliphatic ureas.

2. A process according to claim 1 characterised in that R is a branched or straight chain C₁-C₁₂ alkyl radical.

3. A process according to one of claims 1 and 2 characterised in that R₁ is selected from the following radicals:
branched or straight chain C₁-C₁₈, preferably C₁-C₁₂ alkyl,
branched or straight chain C₂-C₁₈, preferably C₂-C₁₂ alkenyl,
branched or straight chain C₂-C₁₈, preferably C₂-C₁₂ alkynyl,
branched or straight chain C₃-C₁₈, preferably C₃-C₁₂ cycloalkyl,
branched or straight chain C₆-C₁₄, preferably C₆-C₁₀ aryl, and
branched or straight chain C₇-C₁₅, preferably C₇-C₁₁ aralkyl,
said radicals being optionally substituted by one or more halogen atoms or C₁-C₆ alkoxy or alkylthio radicals, wherein the aryl or aralkyl radicals may also comprise in place of one to four carbon atoms, one to four heteroatoms selected from atoms of oxygen, sulphur and nitrogen (for example furyl, thiophenyl and piridyl).

4. A preparation process according to claim 2 characterised in that R₁ is a C₁-C₆ alkyl radical and that R is a C₁-C₆ alkyl radical, preferably methyl.

5. A preparation process according to claim 1 characterised in that:
a) the amides correspond to the following formula:
R₂ CO-NR₃R₄ (III)
wherein R₂ is a hydrogen atom or a C₁-C₆ alkyl or C₃-C₁₀ cycloalkyl group in which from one to three carbon atoms may be respectively replaced by from 1 to 3 heteroatoms selected from the atoms of oxygen, sulphur or nitrogen, R₃ and R₄ which are identical or different are:
a C₁-C₆ alkyl or C₃-C₁₀ cycloalkyl group in which from one to three carbon atoms may be respectively replaced by from one to three heteroatoms selected frcm the atoms of oxygen, sulphur or nitrogen;
b) the lactams corresponding to the formula: 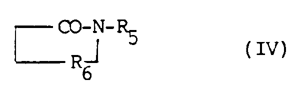 in which R₅ is a C₁-C₆ alkyl or C₃-C₁₀ cycloalkyl group in which from one to three carbon atoms may be respectively replaced by from one to three heteroatoms selected from the atoms of oxygen, sulphur or nitrogen, and R₆ is a C₂-C₁₀ alkylene group in which from one to three carbon atoms may be respectively replaced by from one to three heteroatoms selected from the atans of oxygen, sulphur or nitrogen;
c) the ureas correspond to the formula: 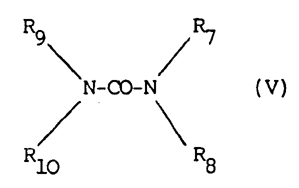 R₇, R₈, R₉ and R₁₀ which are identical or different representing a hydrogen atom or a C₁-C₆ alkyl or C₃-C₁₀ group in which from one to three carbon atoms may be respectively replaced by from one to three heteroatoms selected from the atoms of oxygen, sulphur or nitrogen.

6. A preparation process according to claim 1 characterised in that the reaction is effected in a solvent medium, preferably an aromatic solvent which may or may not be halogenated.

7. A preparation process according to claim 1 characterised in that the COCl₂/lactate molar ratio is between 0.9 and 3 and preferably between 0.95 and 1.5.

8. A preparation process according to claim 1 characterised in that the amides, lactams and ureas/COCl₂ molar ratio is between 1/100 and 1/2, preferably lower than 1/5.

9. A process according to claim 1 characterised in that the reaction takes place in bulk and continuously.

10. A process according to claim 1 characterised in that the temperature of the reaction is between 0°C and 100°C, preferably in the vicinity of 80°C.

## Patentansprüche

1. Verfahren zur Herstellung von optisch aktiven alpha-Chlorcarboxysäureestern der Formel 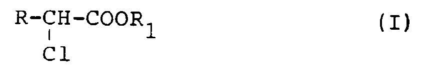 ausgehend von einem optisch aktiven alpha-Hydroxycarboxylsäureester der Formel 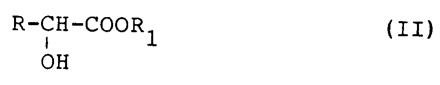 wobei in Formel (I) und (II):
- R ein linearer oder verzweigter C₁-C₁₈-Alkylrest,
- R₁ ein ggf. substituierter Kohlenwasserstoffrest ist,
dadurch gekennzeichnet, daß man den Ester (II) mit Phosgen und einer Verbindung aus der Gruppe der aliphatischen Amide, der aliphatischen Lactame oder der aliphatischen Carbamide umsetzt.

2. Verfahren nach Anspruch 1, dadurch gekennzeichnet, daß R ein linearer oder verzweigter C₁-C₁₂-Alkylrest ist.

3. Verfahren nach einem der Ansprüche 1 oder 2, dadurch gekennzeichnet, daß R₁ aus der Gruppe der folgenden Reste gewählt ist:
linearer oder verzweigter C₁-C₁₈-, vorzugsweise C₁-C₁₂-Alkylrest,
linearer oder verzweigter C₂-C₁₈-, vorzugsweise C₂-C₁₂-Alkenylrest,
linearer oder verzweigter C₂-C₁₈-, vorzugsweise C₂-C₁₂-Alkinylrest,
linearer oder verzweigter C₃-C₁₈-, vorzugsweise C₃-C₁₂-Cycloalkylrest,
linearer oder verzweigter C₆-C₁₄-, vorzugsweise C₆-C₁₀-Arylrest,
linearer oder verzweigter C₇-C₁₅-, vorzugsweise C₇-C₁₁-Aralkylrest,
diese Reste können eventuell durch ein oder mehrere Halogenatome, C₁-C₆-Alkoxy- oder Thioalkylreste substituiert sein, die Aryl- oder Aralkylreste können ein bis 4 Kohlenstoffatome aufweisen, die durch ein bis 4 Heteroatome aus der Gruppe von Sauerstoff, Schwefel und Stickstoff substituierte sind (z.B. Furyl, Thiophenyl, Pyridyl).

4. Verfahren nach Anspruch 2, dadurch gekennzeichnet, daß R₁ ein C₁-C₆ -Alkylrest und R ein C₁-C₆-Alkylrest, vorzugsweise Methyl ist.

5. Verfahren nach Anspruch 1, dadurch gekennzeichnet, daß:
a) die Amide der Formel
R₂CO-NR₃R₄ (III)
entsprechen, in der R₂ ein Wasserstoffatom, eine C₁-C₆-Alkylgruppe oder eine C₃-C₁₀-Cycloalkylgruppe ist, in der ein bis drei Kohlenstoffatome jeweils durch 1 bis 3 Heteroatome aus der Gruppe von Sauerstoff, Schwefel oder Stickstoff ersetzt sein können, R₃ und R₄ gleich oder verschieden, sind: eine C₁-C₆-Alkylgruppe, oder eine C₃-C₁₀-Cycloalkylgruppe, in der ein bis drei Kohlenstoffatome durch jeweils ein bis drei Heteroatome aus der Gruppe von Sauerstoff, Schwefel oder Stickstoff ausgetauscht sein können.
b) die Lactame der Formel 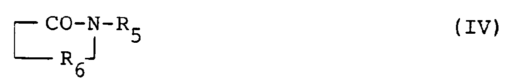 entsprechen, in der R₅ eine C₁-C₆-Alkylgruppe oder eine C₃-C₁₀-Cycloalkylgruppe, in der ein bis drei Kohlenstoffatome jeweils durch ein bis drei Heteroatome aus der Gruppe von Sauerstoff, Schwefel oder Stickstoff ersetzt sein können, und R₆ eine C₂-C₁₀-Alkylengruppe ist, in der ein bis drei Kohlenstoffatome jeweils durch ein bis drei Heteroatome aus der Gruppe von Sauerstoff, Schwefel oder Stickstoff ersetzt sein können.
c) die Carbamide der Formel 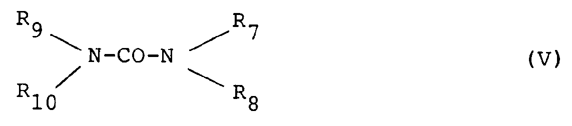 entsprechen, wobei die gleich oder verschiedenen Reste R₇, R₈, R₉ und R₁₀ jeweils ein Wasserstoffatom, eine C₁-C₆-Alkylgruppe oder C₃-C₁₀-Cycloalkylgruppe darstellen, bei der ein bis drei Kohlenstoffatome jeweils durch ein bis drei Heteroatome aus der Gruppe von Sauerstoff, Schwefel oder Stickstoff ersetzt sein können.

6. Verfahren nach Anspruch 1, dadurch gekennzeichnet, daß die Reaktion in einem Lösungsmittel, vorzugsweise in einem ggf. halogenierten, aromatischen Lösungsmittel durchgeführt wird.

7. Verfahren nach Anspruch 1 dadurch gekennzeichnet, daß das molare Verhältnis COCl₂/Lactat zwischen 0,9 und 3, vorzugsweise zwischen 0,95 und 1,5 liegt.

8. Verfahren zur Hestellung nach Anspruch 1, dadurch gekennzeichnet, daß das molare Verhältnis von Amiden, Lactamen und Carbamiden/COCl₂ zwischen 1/100 und 1/2, vorzugsweise unter 1/5 liegt.

9. Verfahren nach Anspruch 1, dadurch gekennzeichnet, daß die Reaktion in Masse und kontinuierlich erfolgt.

10. Verfahren nach Anspruch 1, dadurch gekennzeichnet, daß die Reaktionstemperatur zwischen 0 und 100, vorzugsweise um 80°C liegt.
